# EUROPÄISCHE PATENTSCHRIFT

(11) **EP 3 233 784 B1**
(45) Veröffentlichungstag und Bekanntmachung des Hinweises auf die Patenterteilung: **27.10.2021**
(21) Anmeldenummer: 15817072.0
(22) Anmeldetag: 16.12.2015
(51) Int. Cl.: C07C 69/653, C07C 323/12, C07F 7/18, C09D 4/00

(54) **VERBINDUNGEN MIT FLUORIERTEN ENDGRUPPEN UND DEREN VERWENDUNG IN SCHMUTZABWEISENDEN BESCHICHTUNGEN**
COMPOUNDS WITH FLUORINATED END GROUPS AND THEIR USE IN DIRT-REPELLENT COATINGS
COMPOSÉS AVEC GROUPES TERMINAUX FLUORÉS ET LEUR UTILISATION DANS DES REVÊTEMENTS ANTI-SALISSURES

(30) Priorität: 19.12.2014 EP 14004337
(43) Veröffentlichungstag der Anmeldung: 25.10.2017
(60) Teilanmeldung: 21178677.7
(73) Patentinhaber: Merck Patent GmbH, 64293 Darmstadt (DE)
(72) Erfinder: FRIEDRICH, Reiner, 64342 Seeheim-Jugenheim (DE)
(86) Internationale Anmeldenummer: PCT/EP2015/002530
(87) Internationale Veröffentlichungsnummer: WO 2016/096129

(56) Entgegenhaltungen:
- EP-A2- 0 075 865
- WO-A1-2009/020907
- WO-A1-2010/003931
- WO-A1-2015/050740
- WO-A1-2015/124290
- WO-A2-2010/002623
- JP-A- H01 226 844
- US-A1- 2005 107 645
- US-A1- 2005 113 609
- US-A1- 2009 176 942
- US-A1- 2010 004 478
- CIRKVA V ET AL: "Radical additions to fluoroolefins. Photochemical fluoroalkylation of alkanols and alkane diols with perfluoro vinyl ethers; photo-supported O-alkylation of butane-1,4-diol with hexafluoropropene", JOURNAL OF FLUORINE CHEMISTRY, ELSEVIER, NL, Bd. 80, Nr. 2, 1. Oktober 1996 (1996-10-01), Seiten 135-144, XP004071321, ISSN: 0022-1139, DOI: 10.1016/S0022-1139(96)03514-2
- DATABASE CA [Online] CHEMICAL ABSTRACTS SERVICE, COLUMBUS, OHIO, US; 1953, Knunyants, I.L. et al.: "Addition reactions of fluoroolefins II. Addition of alcohols and thiols to perfluoropropylene", XP002754717, Database accession no. 48:32423 & KNUNYANTS, I.L. ET AL.: "Addition reactions of fluoroolefins II. Addition of alcohols and thiols to perfluoropropylene", IZVESTIYA AKADEMII NAUK SSSR, SERIYA KHIMICHESKAYA, 1953, Seiten 282-289, ISSN: 0002-3353

## Beschreibung

Gegenstand der vorliegenden Erfindung sind neue Verbindungen mit fluorierten Endgruppen und deren Verwendung in z. B. schmutzabweisenden Beschichtungen.

Schmutzabweisende Beschichtungen z. B. in der Displayindustrie bestehen vornehmlich aus perfluorierten Verbindungen, die mittels Siloxangruppen an Oberflächen angebunden werden können. Schmutzabweisende Beschichtungen in der Textilindustrie bestehen vornehmlich aus perfluorierten Verbindungen, die mittels Acrylat, Methacrylat oder Siloxangruppen an Oberflächen angebunden werden können. Aufgrund Ihrer chemischen Stabilität sind diese Verbindungen über die Jahre in Kritik geraten, da der perfluorierte Anteil dieser Materialklasse auf natürlichem Wege nicht abgebaut werden kann. Darüber hinaus ist nicht eindeutig geklärt, welchen Einfluss diese langlebigen Materialien auf die Biosphäre haben und ob sie in unterschiedlichen Tierarten zur Bioaccumulation führen.

US2005/1139609A1, US2009/176942A1, US2005/107645A1, Cirkva et al., J. Fluor. Chem. 1996, 80(2), 135-144, WO2010/0003931 A1, US2010/004478A1, WO2009/020907A1, Knunyants et al., Izvestiya Akademii Nauk SSSR, Seriya Khimicheskaya 1953, 282-289, WO2015/050740A1, WO2015/124290A1, EP0075865A1, JPH01-226844A und WO2010/002623A2 offenbaren fluorierte Verbindungen, aber offenbaren keine Verbindungen wie in der vorliegenden Anmeldung beansprucht oder legen diese nahe.

Es besteht daher Bedarf an alternativen Substanzen für schmutzabweisende Beschichtungen.

Ein erster Gegenstand der vorliegenden Erfindung sind Verbindungen der Formeln (Ib), (Id), (Ie), (Ig) oder (Ih), wobei R"=gleich C1-C4-Alkyl, insbesondere C1- oder C2-Alkyl, R"'= H oder eine Alkylgruppe ist, bevorzugt gleich H oder Methyl ist, 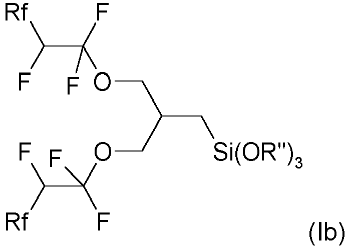 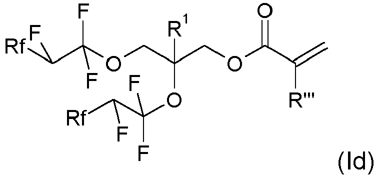 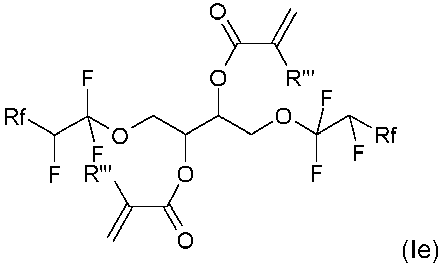 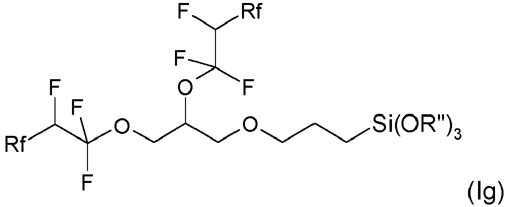 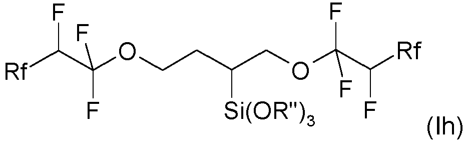 in denen die perfluorierte Gruppe Rf ausgewählt ist aus den Gruppen: CF₃-(CF₂)₁₋₂-, CF₃-(CF₂)₁₋₂-O-, CF₃-O-(CF₂)₁₋₃-, CF₃-O-(CF₂)₁₋₃-O-, CF₃-(CF₂)₁₋₂-O-CF2-, CF₃-O-(CF₂)₁₋₂-O-CF₂-, CF₃-O-(CF₂-O)₁₋₈- und CF₃-O-(CF₂-O)₁₋₈-CF₂-.

In einer Variante kann die perfluorierte Gruppe Rf auch bevorzugt aus den Gruppen CF₃-(CF₂)₁₋₂-, CF₃-(CF₂)₁₋₂-O-, CF₃-O-(CF₂)₁₋₂- und CF₃-O-(CF₂)₁₋₂-O- ausgewählt sein.

Insbesondere solche Verbindungen der Formeln (Ib), (Id), (Ie), (Ig) oder (Ih) sind bevorzugt, in denen Rf eine der bevorzugten oder besonders bevorzugten Gruppen ist und R" = C1- oder C2-Alkyl und/oder R"'= H oder Methyl ist.

Besonders bevorzugt sind hierbei Verbindungen der Formeln (Id), (Ie), (Ig) oder (Ih) insbesondere mit den bevorzugten Rf-, R"-, R"'-und R¹-Gruppen.

Ein Vorteil der neuen Verbindungen ist, dass sie leicht abbaubar sind. Sie weisen gezielte Sollbruchstellen im Molekül auf. So können entsprechende niedermolekulare Bruchstücke entstehen, die atmosphärengängig sind und somit in der Stratosphäre unter UV Licht zersetzt werden können. Hydrofluorether der folgenden Struktur lassen sich z. B. durch Hydrolyse und Oxidation in leicht flüchtige und UV zersetzbare Verbindungen umwandeln. Die Zersetzungsprodukte können dann mit dem Regen aus der Atmosphäre ausgewaschen, in den Boden überführt und dort mineralisiert werden.

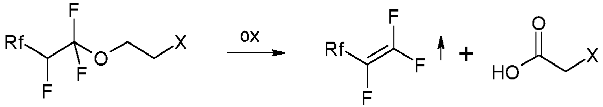

Die Gruppe X stellt dabei eine reaktive Ankergruppe dar, die besonders geeignet ist um z. B. auf Glasoberflächen zu haften (z.B. Trialkoxysilane).

Die Verbindungen der Formeln (Ib), (Id), (Ie), (Ig) oder (Ih) lassen sich leicht synthetisieren.

Die für die Herstellung der Verbindungen verwendeten Ausgangsstoffe sind kommerziell erhältlich und/oder ihre Herstellung ausgehend von kommerziell erhältlichen Edukten ist dem Fachmann geläufig oder sie können in Analogie zu bekannten Syntheseverfahren hergestellt werden, z. B. radikalische Addition siehe: A. A. Il'in et al., Russian Journal of Applied Chemistry, 2007, Vol. 80, No. 3, pp. 405-418.

Die bevorzugten Verbindungen der Formel (Ib), (Ig) und (Ih), die der Substanzklasse der Organosilane angehören, lassen sich durch folgende einfache Synthese realisieren, wie für die untenstehenden nicht beanspruchten Methoxysilane beispielhaft gezeigt ist:

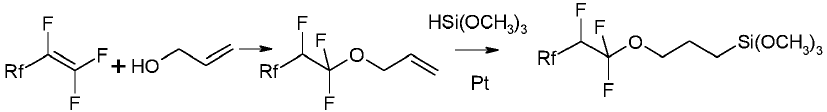

Um beanspruchte verzweigte Hydrofluorethersilane zu erhalten, werden die Perfluorolefine mit bifunktionellen Hydroxyalkenen umgesetzt.

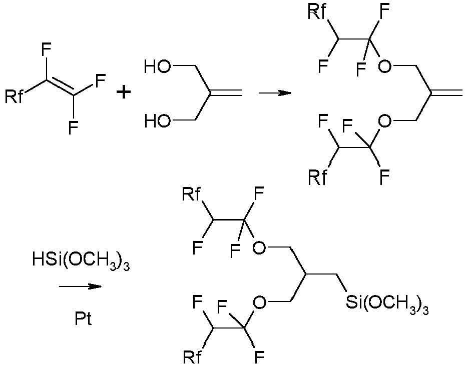

Die Trimethoxysilane können dann einfach in einem geeigneten Lösungsmittel dispergiert werden und auf die zu behandelnde Oberfläche, z. B. Glas, aufgebracht werden. Durch Luftfeuchtigkeit hydrolysiert das Trimethoxysilan und bildet z. B. mit den SiOH Gruppen des Glases eine kovalente dauerhafte Verbindung.

Erfindungsgemäße Verbindungen mit Acrylat- oder Methacrylatgruppen können z. B. durch Umsetzung der entsprechenden Alkohole mit den Säuren oder Säureanhydriden nach dem Fachmann bekannten Methoden hergestellt werden.

Der Abbau der Verbindungen der Formeln (Ib), (Id), (Ie), (Ig) oder (Ih) kann bevorzugt durch ein Verfahren zum Abbau von fluorhaltigen Verbindungen umfassend die folgenden Schritte erfolgen:
a) biologische und/oder abiotischer Abbau des Kohlenstoffgerüsts der fluorhaltigen Verbindungen unter Bildung von, vorzugsweise nicht toxischen, fluorhaltigen Verbindungen, bevorzugt mit einem ausreichend hohen Dampfdruck,
b) Überführen der in Schritt a) gebildeten fluorhaltigen Verbindungen in eine Gasphase,
c) Abbau der in Schritt a) gebildeten fluorhaltigen zu niedermolekularen Verbindungen durch UV-Bestrahlung in der Gasphase,
d) Überführen der in Schritt c) gebildeten niedermolekularen Verbindungen aus der Gasphase in eine flüssige und/oder feste Phase,
e) Mineralisierung der Schritt c) gebildeten niedermolekularen Verbindungen der flüssigen und/oder festen Phase.

Bevorzugt werden in Schritt a) keine fluorhaltigen, Salze gebildet. Insbesondere werden in Schritt a) keine perfluorierten Verbindungen gebildet. Bevorzugt weisen die in Schritt a) gebildeten fluorhaltigen Verbindungen einen ausreichend hohen Dampfdruck auf, um leicht in die Gasphase überzugehen oder überführt werden zu können, bevorzugt bei Normaldruck.

Die erfindungsgemäßen Verbindungen können alleine oder als Gemisch, auch mit anderen fluorierten und/oder nicht fluorierten Verbindungen, verwendet werden, insbesondere zur Herstellung von funktionellen Überzügen und Oberflächenmodifikationen aller Art auf Gegenständen sowohl für Innen- wie auch für Außenbereiche.

Prinzipiell können alle Oberflächen beschichtet werden, insbesondere Glas, Keramik, Emaille, Metalle, Kunststoffe, Elastomere, Naturstoffe, Textilien, gegebenenfalls nach einer geeigneten Vorbehandlung.

Ein weiterer Gegenstand der vorliegenden Erfindung ist die Verwendung der erfindungsgemäßen Verbindungen der Formeln (Ib), (Id), (Ie), (Ig) oder (Ih), und den vorstehend beschriebenen bevorzugten Ausführungsformen zur Herstellung von, z. B. schmutzabweisenden und/oder hydrophoben, Beschichtungen, insbesondere auch zur Textilausrüstung und Glasbeschichtung, z. B. von Beschichtungen in der Displayindustrie.

Neben den Verbindungen der Formeln (Ib), (Id), (Ie), (Ig) oder (Ih), können die Beschichtungen auch Lösemittel, Additive, Tenside, Hilfs- und Füllstoffe enthalten. Beispielhaft seien auch Silikonpartikel und, ggf. oberflächenmodifizierte, Pigmente genannt.

Bevorzugte Einsatzgebiete sind beispielsweise die Verwendung der erfindungsgemäßen Verbindungen in Beschichtungen für optische Elemente oder Textilien, wie z. B. die Verwendung in Antifingerprint Coatings, z. B. für Displays, optische Linsen, Brillengläser, Objektive für Kameras, Ferngläser, Fensterscheiben oder Spiegel, oder als Hydrophobiermittel zur Textilausrüstung.

Die Aufbringung der erfindungsgemäßen Verbindungen oder sie enthaltenden Mischungen auf eine geeignete Oberfläche kann, vollflächig oder teilflächig, durch verschiedene dem Fachmann bekannte Beschichtungsprozesse erfolgen, z. B. mittels CVD-, PVD-, Spray-Coating-, Ink-Jet-, Offset-Prozessen.

Alle hier genannten Verwendungen erfindungsgemäß einzusetzender Verbindungen sind Gegenstand der vorliegenden Erfindung. Die jeweilige Anwendung von Verbindungen der Formeln (Ib), (Id), (Ie), (Ig) oder (Ih), zu den genannten Zwecken ist dem Fachmann bekannt, so dass der Einsatz der erfindungsgemäß einzusetzenden Verbindungen keine Probleme bereitet.

Gegenstand der Erfindung sind auch Mittel, in denen mindestens eine der erfindungsgemäßen Verbindungen enthalten ist, wobei die Mittel auch Lösemittel, Additive, Tenside, Hilfs- und Füllstoffe enthalten können Gegenstand der Erfindung sind auch beschichtete Gegenstände, insbesondere die vorstehend genannten Gegenstände, deren Beschichtung unter Verwendung von mindestens einer erfindungsgemäßen Verbindung hergestellt wurde. Bevorzugt sind Displays, optische Linsen, Brillengläser, Objektive für Kameras, Ferngläser, Fensterscheiben, Spiegel und Textilien.

Die folgenden Beispiele erläutern die vorliegende Erfindung näher, ohne den Schutzbereich zu beschränken.

### Beispiele

### Abkürzungen

- TEMPO: 2,2,6,6-Tetramethylpiperidinyloxyl
- THF: Tetrahydrofuran
- MTBE: tert-Butylmethylether
- RT: Raumtemperatur

### Beispiel 1: Synthese einer Verbindung der Formel (Id) mit R¹ = H, R'" = CH₃ und Rf = C₃F₇-O

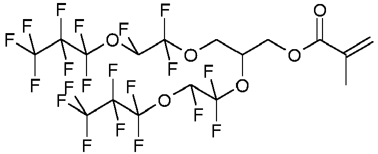

### Beispiel 1a:

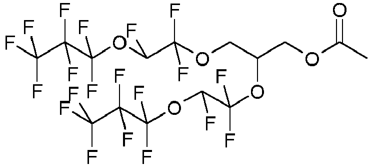

100g 2,3-Dihydroxy-propylacetat, 595 g Perfluorpropylvinylether, 134 g Kaliumcarbonat und 460 g Acetonitril werden in einem Druckbehälter bei 80°C 70 Std gerührt. Der Innendruck steigt während der Reaktion auf 4 bar. Das Reaktionsprodukt wird mit 100 ml Acetonitril aus dem Reaktor gewaschen, das Gemisch wird filtriert und das Lösungsmittel am Rotationsverdampfer abgetrennt und das Rohprodukt bei vermindertem Druck (Sdp 75 °C bei 0,3 mbar) destilliert. Ausbeute: 533 g = 80%. 1H-NMR: 6,8 ppm (m, 2 H, -CFH); 4,8 ppm (m,1 H, -OCH); 4,3 ppm (m, 4H, -CH2); 2,1 ppm (s, 3 H, O=CCH3)

### Beispiel 1b:

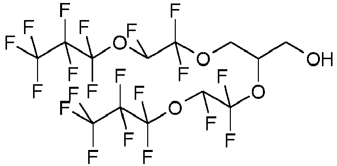

30 g Ester werden mit 20 ml Methanol, 0,5 g Natriummethylat, 27 mg Ru-MACHO (Takasago International, JP) für 12 Std bei 50 bar und 40 °C in einem Druckreaktor mit Wasserstoff hydriert. Das Reaktionsgemisch wird mit 50 ml Methyl-tert.butylether und 50 ml Wasser versetzt, die organische Phase abgetrennt und über NaSO4 getrocknet. Das Produkt wird vom Lösungsmittel befreit und im Vakuum destilliert (Sdp.: 74°C bei 0,4 mbar). Ausbeute: 20 g = 70%).
1H-NMR: 6,8 ppm (m, 2 H, -CFH); 4,8 - 4,3 ppm (m, 5H, -CH2);

### Beispiel 1c:

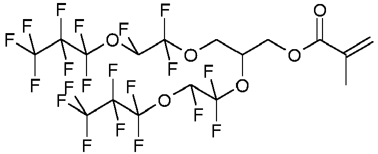

10 g Fluoralkoholalkohol werden mit 0,5 g Toluol-4-sulfonsäure-Monohydrat in 35 ml Toluol gelöst. Dann werden unter Rühren 2,7 g Methacrylsäureanhydrid langsam zugegeben und die Reaktionsmischung für 24h bei 110 °C gerührt. Der auf Raumtemperatur abgekühlte Ansatz wird mit 25 mL Wasser und 25 mL MTBE versetzt und im Scheidetrichter abgetrennt. Anschließend wird die wässrige Phase zweimal mit 25 mL MTBE gewaschen. Die vereinigte organische Phase wird Natriumsulfat getrocknet und filtriert. Anschließend das Lösungsmittel am Rotationsverdampfer destilliert. Ausbeute: 9,98g
1H-NMR: 6,8 ppm (m, 2 H, -CFH); 6,0 ppm (d,1H, =CH); 5,6 ppm (d, 1H,=CH); 4,8 - 4,3 ppm (m, 5H, -CH2); 1,7 ppm (s, 3H, -CH3)

### Vergleichsbeispiel 2: Synthese einer Verbindung der Formel (I'e) mit R'" = CH₃ und Rf = C₃F₇-O

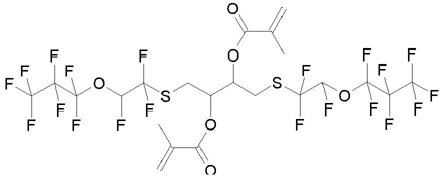

### Beispiel 2a:

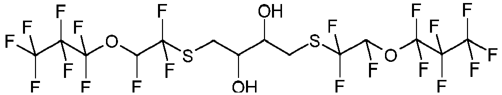

8,63 g Perfluorpropylvinylether werden mit 2,5g 1,4-Dimercaptobutane-2,3-diol, 25 ml Acetonitril und 0,67 g Kaliumcarbonat in einem Druckreaktor für 18 Std auf 120°C erhitzt. Das Reaktionsgemisch wird mit 25 ml Wasser und 25 ml MTBE versetzt und die Phasen getrennt. Die wässrige Phase wird mit 2x25mL MTBE extrahiert und die vereinigte organische Phase mit 70mL Wasser und 70mL gesättigter NaCl-Lösung gewaschen. Das Extrakt wird über Natriumsulfat getrocknet und das Lösemittel destilliert. Ausbeute: 9,82g = 88%
1H-NMR: 6,8 ppm (d, 2 H, -CFH); 3,6 ppm (m, 2H, -CHOH); 3,0 ppm (m, 4H, -SCH2);

### Beispiel 2b:

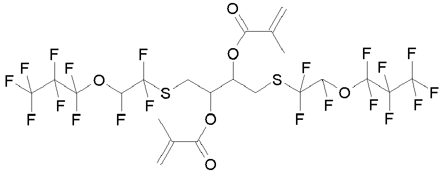

10 g Fluoralkoholalkohol werden mit 0,5 g Toluol-4-sulfonsäure-Monohydrat in 35 ml Toluol gelöst. Dann werden unter Rühren 4,5 g Methacrylsäureanhydrid langsam zugegeben und die Reaktionsmischung für 24h bei 110 °C gerührt. Der auf Raumtemperatur abgekühlte Ansatz wird mit 25 mL Wasser und 25 mL MTBE versetzt und im Scheidetrichter abgetrennt. Anschließend wird die wässrige Phase zweimal mit 25 mL MTBE gewaschen. Die vereinigte organische Phase wird Natriumsulfat getrocknet und filtriert. Anschließend das Lösungsmittel am Rotationsverdampfer destilliert. Ausbeute: 14,5 g
1H-NMR: 6,8 ppm (m, 2 H, -CFH); 6,0 ppm (d,2H, =CH); 5,6 ppm (d,2H,=CH); 3,6 ppm (m, 2H, -OCH-); 3,0 ppm (m, 4H, -SCH2); 1,6 ppm (s, 6H, -CH3)

### Vergleichsbeispiel 3: Synthese einer Verbindung der Formel (I'c) mit R'" = CH₃ und Rf = C₃F₇-O

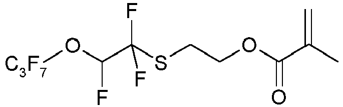

### Vergleichsbeispiel 3a:

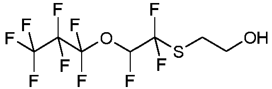

77,30 g Perfluorpropylvinylether werden mit 52,21 g 2-Mercaptoethanol 40 ml Acetonitril und 12 g Kaliumcarbonat in einem Druckreaktor für 18 Std auf 100°C erhitzt. Das Reaktionsgemisch wird mit 25 ml Wasser und 25 ml MTBE versetzt und die Phasen getrennt. Die wässrige Phase wird mit 2x25mL MTBE extrahiert und die vereinigte organische Phase mit 70mL Wasser und 70mL gesättigter NaCl-Lösung gewaschen. Das Extrakt wird über Natriumsulfat getrocknet, das Lösemittel entfernt und das Rohmaterial destilliert (Sdp 45-49 °C bei 0,1 mbar). Ausbeute: 63,40g = 63% 1H-NMR: 6,8 ppm (dt, 1 H, -CFH); 3,6 ppm (t, 2H, -CH2O-); 3,1 ppm (t, 2H, -SCH2);

### Vergleichsbeispiel 3b:

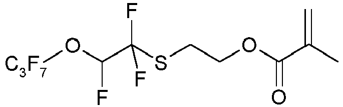

10 g Fluoralkoholalkohol werden mit 0,5 g Toluol-4-sulfonsäure-Monohydrat in 60 ml Toluol gelöst. Dann werden unter Rühren 4,5 g Methacrylsäureanhydrid langsam zugegeben und die Reaktionsmischung für 24h bei 110 °C gerührt. Der auf Raumtemperatur abgekühlte Ansatz wird mit 25 mL Wasser und 25 mL MTBE versetzt und im Scheidetrichter abgetrennt. Anschließend wird die wässrige Phase zweimal mit 25 mL MTBE gewaschen. Die vereinigte organische Phase wird Natriumsulfat getrocknet, filtriert und das Lösungsmittel am Rotationsverdampfer entfernt. Ausbeute: 11,30 g = 94%
1H-NMR: 7,1 ppm (m, 1 H, -CFH); 6,1 ppm (m,1H, =CH); 5,7 ppm (m,1H,=CH); 4,3 ppm (t, 2H, -CH2O-); 3,2 ppm (t,2H, -SCH2); 1,9 ppm (s, 3H, -CH3)

### Beispiel 4: Synthese einer Verbindung der Formel (Id) mit R" = CH₃, R¹ = CH₃ und Rf = CF₃-O-C₃F₆-O

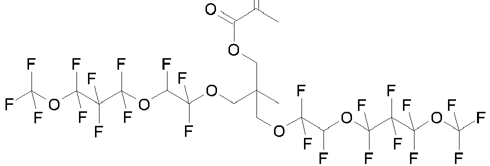

### Beispiel 4a:

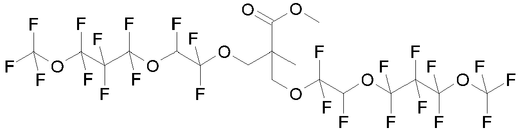

5 g 3-Hydroxy-2-hydroxymethyl-2-methylpropionsäuremethylester, 20 ml 1,1,2,2,3,3-Hexafluoro-1-trifluoromethoxy-3-trifluorovinyloxypropan 60 ml Acetonitril und 6 g Kaliumcarbonat werden in einem Druckreaktor bei 80°C 20 h gerührt. Es stellte sich ein Druck von 1,5bar ein. Die Reaktion wird abgebrochen und das Reaktionsgemisch mit Wasser und MTBE versetzt. Die Phasen werden getrennt und die wässrige Phase mit 2x50mL MTBE extrahiert. Anschließend wird die vereinigte organische Phase mit 70mL Wasser und 70mL NaCl-Lösung gewaschen. Das Extrakt wird über Natriumsulfat getrocknet und das Lösemittel destilliert.
Auswaage: 20,58g 75%
1H-NMR: 6,6 ppm (m, 2 H, -CFH); 4,2 ppm (dd, 4H, -CH2O); 3,7 ppm (s,3H, -OCH3); 1,25 ppm (s, 3H CCH3)

### Beispiel 4b:

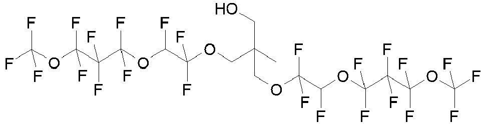

20 g Ester werden mit 15 ml Methanol, 0,3 g Natriummethylat, 15 mg Katalysator für 12 Std bei 50 bar und 40 °C in einem Druckreaktor mit Wasserstoff hydriert. Das Reaktionsgemisch wird mit 50 ml Methyl-tert.butylether und 50 ml Wasser versetzt, die organische Phase abgetrennt und über NaSO4 getrocknet. Das Produkt wird vom Lösungsmittel befreit und im Vakuum destilliert (Sdp.: 95°C bei 0,1 mbar). Ausbeute: 16 g = 82%).
1H-NMR: 6,6 ppm (m, 2 H, -CFH); 6,1 ppm (m,1H, =CH); 5,7 ppm (m,1H,=CH); 4,3-4,1 ppm (m, 6H, -CH2O); 1,3 ppm (s, 3H CCH3)

### Beispiel 4c:

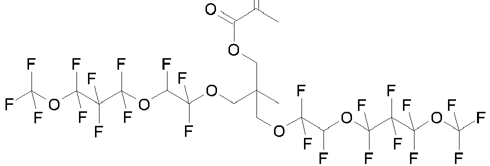

10 g Fluoralkoholalkohol werden mit 0,5 g Toluol-4-sulfonsäure-Monohydrat in 35 ml Toluol gelöst. Dann werden unter Rühren 2 g Methacrylsäureanhydrid langsam zugegeben und die Reaktionsmischung für 24h bei 110 °C gerührt. Der auf Raumtemperatur abgekühlte Ansatz wird mit 25 mL Wasser und 25 mL MTBE versetzt und im Scheidetrichter abgetrennt. Anschließend wird die wässrige Phase zweimal mit 25 mL MTBE gewaschen und die vereinigte organische Phase wird mit Natriumsulfat getrocknet und filtriert. Das Lösungsmittel wird am Rotationsverdampfer entfernt. Ausbeute: 10.5 g = 96%
1H-NMR: 6,6 ppm (m, 2 H, -CFH); 6,2 ppm (m,1H, =CH); 5,7 ppm (m,1H,=CH); 4,3-4,1 ppm (m, 6H, -CH2O); 1,9 ppm (s, 3H, -CH3); 1,25 ppm (s, 3H CCH3)

### Beispiel 5: Synthese einer Verbindung der Formel (Ih) mit R" = CH₃ und Rf = C₃F₇-O

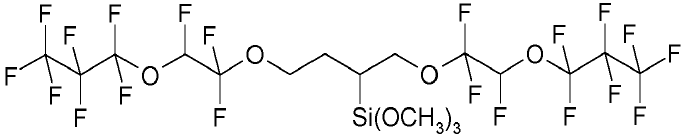

### Beispiel 5a:

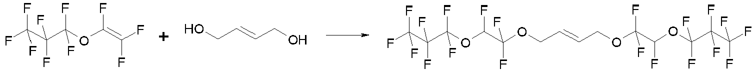

3,56 g But-2-en1,4-diol und 26,87 g Perfluorovinyloxypropan werden mit 2,84 g KOH und 27,5 g Acetonitril in einem Autoklaven auf 80°C erhitzt und 24 h bei dieser Temperatur zur Reaktion gebracht. Der Innendruck fällt dabei von anfangs 2,6 bar auf 1 bar. Der Ansatz wird nach Abkühlen auf Raumtemperatur filtriert und das Lösungsmittel destilliert. Das Lösemittel wird im Vakuum entfernt. Rohausbeute Ausbeute: m=30,28g Das Rohprodukt wird in 30 ml Acetonitril gelöst, mit 20 ml Toluol versetzt, wodurch ein weißes Gel ausfällt. Der Niederschlag wird über wenig Kieselgel abfiltriert und mit 20 ml Acetonitril nachgewaschen. Nach Entfernung des Lösungsmittels wird der Rückstand getrocknet. Ausbeute: m= 24,8g
1H-NMR: 6,7 ppm (m, 2 H, -CFH); 6,0 ppm (s,2H, =CH); 4,6 ppm (s, 4H, - CH2O);

### Beispiel 5b:

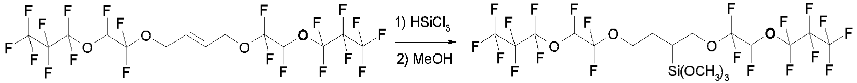

In einem 100mL 4-Halskolben werden unter Eiskühlung und unter Argon 0,15 g Hexachloroplatin (IV)-säure- Hexahydrat (~40%Pt) und 10 g Perfluoroolefin in 12 ml abs. THF vorgelegt. Unter Rühren und Eiskühlung werden 1,84 ml Trichlorsilan über eine Spritze zum Ansatz zugegeben, so dass die IT 5°C nicht überschreitet. Der Ansatz auf 60°C geheizt und bei dieser Temperatur 4h zur Reaktion gebracht. Anschließend wird unter Argon über Nacht auf RT abgekühlt. Es wird mit 5mL Trimethylorthoformiat versetzt und anschließend 2mL MeOH zugegeben. Die Temperatur steigt dabei um 5K auf 33°C. Es wird auf 50°C erwärmt und 2h bei dieser Temperatur gerührt. Der Ansatz wird abgekühlt, das Lösemittel entfernt und der Rückstand im Vakuum getrocknet. Rohausbeute: m=18,95g Der Ansatz wird bei 120 °C in einer Kugelrohrdestille im Hochvakuum aufgereinigt.
1H-NMR: 6,7 ppm (dt, 2 H, -CFH); 3,8 ppm (m,4 H, -OCH2); 3,5 ppm (s,9H, -OCH3); 1,4 ppm (m,2 H, -CH2); 0,6 ppm (m,1 H, -SiCH);

### Beispiel 6: Synthese einer Verbindung der Formel (Ig) mit R" = CH₃ und Rf = C₃F₇-O

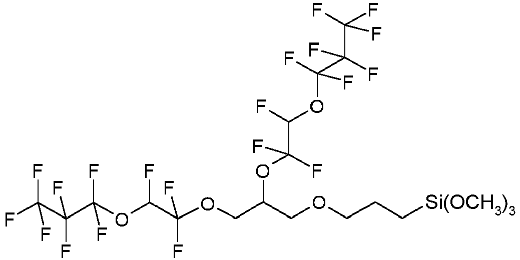

### Beispiel 6a:

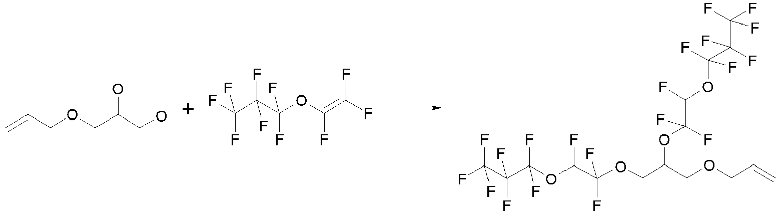

10,0 g Allyloxy-1,2-propandiol werden mit 60,4 g Perfluorovinyloxypropane, 13,6 g Kaliumcarbonat und 46,5 g Acetonitril im Autoklaven bei 80°C für 48h zur Reaktion gebracht. Nach Beendigung wird der Ansatz filtriert und das Lösemittel im Vakuum entfernt. Ausbeute: m=50,2g Das Produkt wird im Vakuum destilliert (64-65°C bei 3 mbar)
1H-NMR: 6,8 ppm (d, 2 H, -CFH); 5,9 ppm (m, 1H, CH); 5,3 ppm (d, 1H, =CH₂), 5,2 ppm (d, 1H, =CH₂), 4,8 ppm (d, 1H, =CH) 4,2 ppm (m,2 H, - OCH₂); 4,0 ppm (m,2 H, -OCH₂); 3,8 ppm (m,2 H, -OCH₂)

### Beispiel 6b:

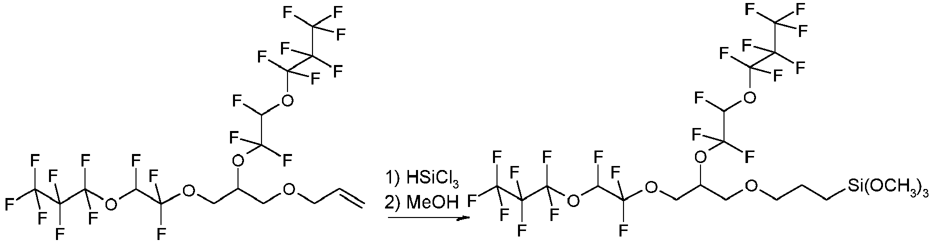

In einem 100mL 4-Halskolben werden unter Eiskühlung und unter Argon 0,15 g Hexachloroplatin (IV)-säure- Hexahydrat (~40%Pt) und 10 g Perfluoroolefin in 12 ml abs. THF vorgelegt. Unter Rühren und Eiskühlung werden 1,84 ml Trichlorsilan über eine Spritze zum Ansatz zugegeben so dass die IT 5°C nicht überschreitet. Der Ansatz auf 60°C geheizt und bei dieser Temperatur 4h zur Reaktion gebracht. Anschließend wird unter Argon über Nacht auf RT abgekühlt. Es wird mit 5mL Trimethylorthoformiat versetzt und anschließend 2mL MeOH zugegeben. Es wird auf 50°C erwärmt und 6h bei dieser Temperatur gerührt. Der Ansatz wird abgekühlt, das Lösemittel entfernt und der Rückstand im Vakuum getrocknet. Rohausbeute: m=15,2g

Das Produkt wird in der Kugelrohrdestille aufgereinigt. 115°@ C 0,01 mbar 1H-NMR: 6,8 ppm (d, 2 H, -CFH); 5,9 ppm (m, 1H, CH); 4,2 ppm (m, 2H, - OCH₂); 4,0 ppm (m, 2H, -OCH₂); 3,5 ppm (s, 9H,-OCH₃); 3,4 ppm (m, 2H, - OCH₂) 1,5-0,6ppm (m, 4H, CH₂-CH₂-Si-)

## Patentansprüche

1. Verbindungen,
**dadurch gekennzeichnet, dass** sie einer der Formeln (Ib), (Id), (Ie), (Ig) oder (Ih) entsprechen 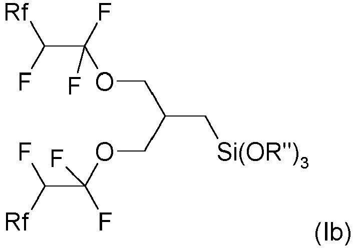 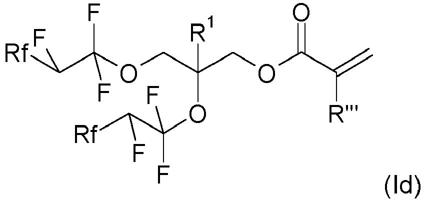 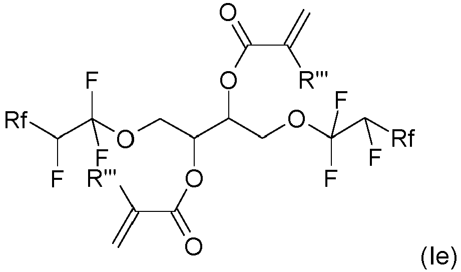 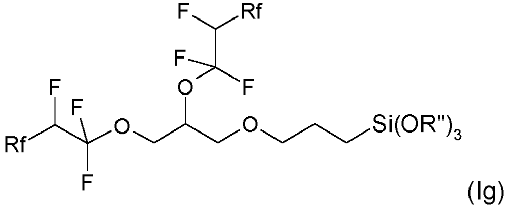 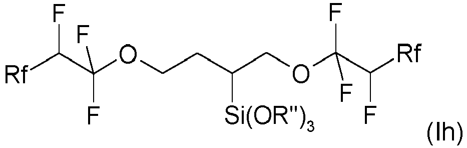 wobei die Gruppe Rf aus den Gruppen CF₃-(CF₂)₁₋₂-, CF₃-(CF₂)₁₋₂-O-, CF₃-O-(CF₂)₁₋₃-, CF₃-O-(CF₂)₁₋₂-O-, CF₃-(CF₂)₁₋₂-O-CF₂-, CF₃-O-(CF₂)₁₋₂-O-CF₂-, CF₃-O-(CF₂-O)₁₋₈- und CF₃-O-(CF₂-O)₁₋₈-CF₂-ausgewählt ist, R"=gleich C1-C4-Alkyl und R'" = H oder eine Alkylgruppe ist.

2. Verbindungen gemäß Anspruch 1, **dadurch gekennzeichnet, dass** die Gruppe R gleich H oder C1-C3 Alkyl ist.

3. Verbindungen gemäß einem oder mehreren der Ansprüche 1 und 2, **dadurch gekennzeichnet, dass**
die Gruppe R'" gleich H oder eine Methylgruppe ist.

4. Verbindungen gemäß einem oder mehreren der Ansprüche 1 bis 3, **dadurch gekennzeichnet, dass** R" gleich C1-oder C2-Alkyl ist.

5. Verbindungen gemäß einem oder mehreren der Ansprüche 1 bis 4, **dadurch gekennzeichnet, dass**
Rf gleich CF₃-(CF₂)₁₋₂-, CF₃-(CF₂)₁₋₂-O-, CF₃-O-(CF₂)₁₋₃- oder CF₃-O-(CF₂)₁₋₂-O- ist.

6. Verwendung von Verbindungen gemäß einem oder mehreren der Ansprüche 1 bis 5 zur Herstellung von funktionellen Überzügen und Oberflächenmodifikationen, insbesondere von schmutzabweisenden Beschichtungen.

7. Verfahren zum Abbau von fluorhaltigen Verbindungen gemäß einem oder mehreren der Ansprüche 1 bis 5 umfassend die folgenden Schritte:
a) biologischer und/oder abiotischer Abbau des Kohlenstoffgerüsts der fluorhaltigen Verbindungen unter Bildung von fluorhaltigen Verbindungen,
b) Überführen der in Schritt a) gebildeten fluorhaltigen Verbindungen in eine Gasphase,
c) Abbau der in Schritt a) gebildeten fluorhaltigen Verbindungen zu niedermolekularen Verbindungen durch UV-Bestrahlung in der Gasphase,
d) Überführen der in Schritt c) gebildeten niedermolekularen Verbindungen aus der Gasphase in eine flüssige und/oder feste Phase,
Mineralisierung der in Schritt c) gebildeten niedermolekularen Verbindungen in der Flüssigen und/oder festen Phase.

8. Mittel enthaltend mindesten eine Verbindung gemäß einem oder mehreren der Ansprüche 1 bis 5 und einen für den jeweiligen Verwendungszweck geeigneten Träger sowie ggf. weitere Additive.

9. Beschichteter Gegenstand, dessen Beschichtung unter Verwendung von mindestens einer Verbindung gemäß einem oder mehreren der Ansprüche 1 bis 5 hergestellt wurde.

## Claims

1. Compounds,
**characterised in that** they conform to one of the formulae (Ib), (Id), (Ie), (Ig) or (Ih) 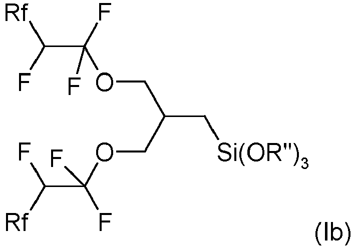 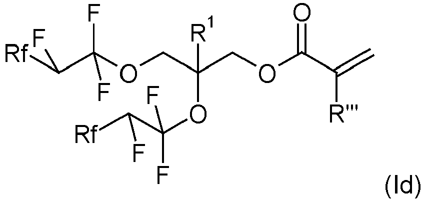 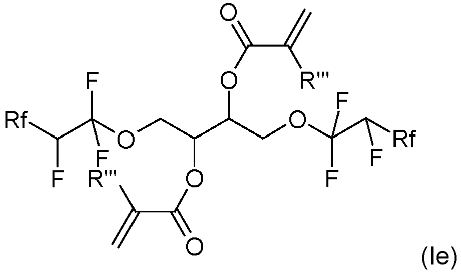 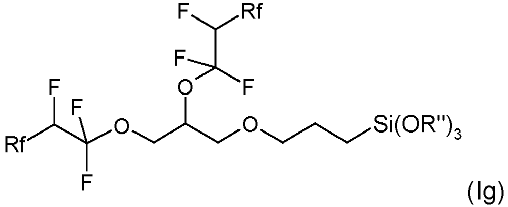 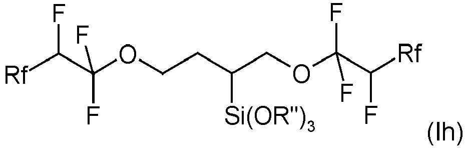 where the group Rf is selected from the groups CF₃-(CF₂)₁₋₂-, CF₃-(CF₂)₁₋₂-O-, CF₃-O-(CF₂)₁₋₃-, CF₃-O-(CF₂)₁₋₂-O-, CF₃-(CF₂)₁₋₂-O-CF₂-, CF₃-O-(CF₂)₁₋₂-O-CF₂-, CF₃-O-(CF₂-O)₁₋₈- and CF₃-O-(CF₂-O)₁₋₈-CF₂-, R"= C1-C4-alkyl and R'" = H or an alkyl group.

2. Compounds according to Claim 1, **characterised in that** the group R is equal to H or C1-C3 alkyl.

3. Compounds according to one or more of Claims 1 and 2, **characterised in that**
the group R'" is equal to H or a methyl group.

4. Compounds according to one or more of Claims 1 to 3, **characterised in that** the group R" is equal to C1- or C2-alkyl.

5. Compounds according to one or more of Claims 1 to 4, **characterised in that**
Rf is equal to CF₃-(CF₂)₁₋₂-, CF₃-(CF₂)₁₋₂-O-, CF₃-O-(CF₂)₁₋₃- or CF₃-O-(CF₂)₁₋₂-O-.

6. Use of compounds according to one or more of Claims 1 to 5 for the production of functional coatings and surface modifications, in particular of dirt-repellent coatings.

7. Process for the degradation of fluorine-containing compounds according to one or more of Claims 1 to 5 comprising the following steps:
a) biological and/or abiotic degradation of the carbon skeleton of the fluorine-containing compounds with formation of fluorine-containing compounds,
b) conversion of the fluorine-containing compounds formed in step a) into a gas phase,
c) degradation of the fluorine-containing compounds formed in step a) into low-molecular-weight compounds by UV irradiation in the gas phase,
d) conversion of the low-molecular-weight compounds formed in step c) from the gas phase into a liquid and/or solid phase, mineralisation of the low-molecular-weight compounds formed in step c) in the liquid and/or solid phase.

8. Composition comprising at least one compound according to one or more of Claims 1 to 5 and a support which is suitable for the respective application and optionally further additives.

9. Coated article whose coating has been produced using at least one compound according to one or more of Claims 1 to 5.

## Revendications

1. Composés,
**caractérisés en ce qu'**ils répondent à l'une des formules (Ib), (Id), (le), (Ig) ou (Ih) 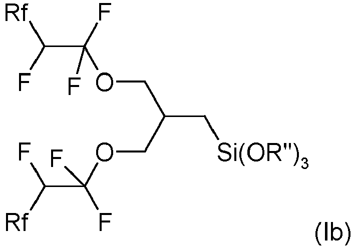 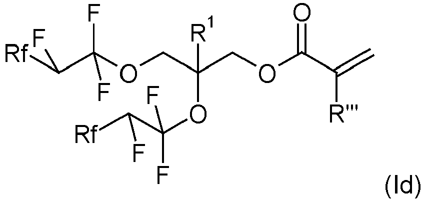 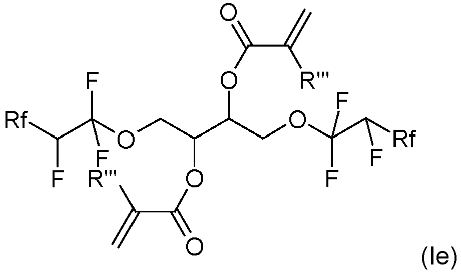 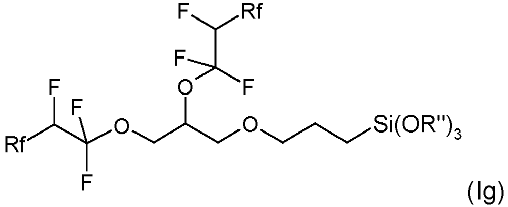 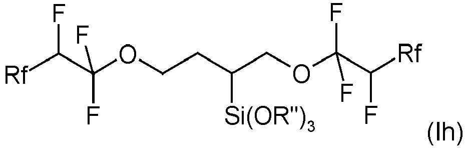 où le groupement Rf est choisi parmi les groupements CF₃-(CF₂)₁₋₂-, CF₃-(CF₂)₁₋₂-O-, CF₃-O-(CF₂)₁₋₃-, CF₃-O-(CF₂)₁₋₂-O-, CF₃-(CF₂)₁₋₂-O-CF₂-, CF₃-O-(CF₂)₁₋₂-O-CF₂-, CF₃-O-(CF₂-O)₁₋₈- et CF₃-O-(CF₂-O)₁₋₈-CF₂-, R"= C1-C4-alkyle et R'" = H ou un groupement alkyle.

2. Composés selon la revendication 1, **caractérisés en ce que** le groupement R est égal à H ou C1-C3 alkyle.

3. Composés selon l'une ou plusieurs parmi les revendications 1 et 2, **caractérisés en ce que**
le groupement R'" est égal à H ou un groupement méthyle.

4. Composés selon l'une ou plusieurs parmi les revendications 1 à 3, **caractérisés en ce que** le groupement R" est égal à C1- ou C2-alkyle.

5. Composés selon l'une ou plusieurs parmi les revendications 1 à 4, **caractérisés en ce que**
Rf est égal à CF₃-(CF₂)₁₋₂-, CF₃-(CF₂)₁₋₂-O-, CF₃-O-(CF₂)₁₋₃- ou CF₃-O-(CF₂)₁₋₂-O-.

6. Utilisation de composés selon l'une ou plusieurs parmi les revendications 1 à 5, pour la production de revêtements fonctionnels et de modifications de surface, en particulier de revêtements anti-salissures.

7. Procédé de dégradation de composés contenant du fluor selon l'une ou plusieurs parmi les revendications 1 à 5, comprenant les étapes suivantes :
a) la dégradation biologique et/ou abiotique du squelette carboné des composés contenant du fluor, avec la formation de composés contenant du fluor,
b) la conversion des composés contenant du fluor, formés dans l'étape a), en une phase gazeuse,
c) la dégradation des composés contenant du fluor, formés dans l'étape a), en composés de bas poids moléculaire par rayonnement UV dans la phase gazeuse,
d) la conversion des composés de bas poids moléculaire, formés dans l'étape c), de la phase gazeuse en une phase liquide et/ou solide,
la minéralisation des composés de bas poids moléculaire, formés dans l'étape c), dans la phase liquide et/ou solide.

8. Composition comprenant au moins un composé selon l'une ou plusieurs parmi les revendications 1 à 5 et un support qui est convenable pour l'application respective, et éventuellement d'autres additifs.

9. Article revêtu, dont le revêtement a été produit en utilisant au moins un composé selon l'une ou plusieurs parmi les revendications 1 à 5.
